# EUROPÄISCHE PATENTSCHRIFT

(11) **EP 1 392 314 B1**
(45) Veröffentlichungstag und Bekanntmachung des Hinweises auf die Patenterteilung: **20.12.2006**
(21) Anmeldenummer: 02740570.3
(22) Anmeldetag: 07.05.2002
(51) Int. Cl.: A61K 31/53, A61P 25/28

(54) **NEUE VERWENDUNG VON 2-[2-Ethoxy-5-(4-methyl-piperazin-1-sulfonyl)-phenyl]-5-methyl-7-propyl-3H-imidazo[5,1-f][1,2,4]triazin-4-on**
NOVEL USE OF 2-[2-Ethoxy-5-(4-methyl-piperazin-1-sulfonyl)-phenyl]-5-methyl-7-propyl-3H-imidazo[5,1-f][1,2,4]triazin-4-on
NOUVELLE UTILISATION DU 2-[2-Ethoxy-5-(4-methyl-piperazin-1-sulfonyl)-phenyl]-5-methyl-7-propyl-3H-imidazo[5,1-f][1,2,4]triazin-4-on

(30) Priorität: 09.05.2001 DE 10122576; 30.05.2001 DE 10126198
(43) Veröffentlichungstag der Anmeldung: 03.03.2004
(73) Patentinhaber: Bayer HealthCare AG, 51368 Leverkusen (DE)
(72) Erfinder: NIEWÖHNER, Ulrich, deceased (DE); BISCHOFF, Erwin, 42115 Wuppertal (DE); HANING, Helmut, 42115 Wuppertal (DE); RAHBAR, Afssaneh, 50226 Frechen (DE); BANDEL, Tiemo-Joerg, 46145 Oberhausen (DE); BARTH, Wolfgang, 42349 Wuppertal (DE); VAN DER STAAY, Franz-Josef, 8251 BB Dronten (NL)
(86) Internationale Anmeldenummer: PCT/EP2002/005002
(87) Internationale Veröffentlichungsnummer: WO 2002/089808

(56) Entgegenhaltungen:
- WO-A-01/27101
- WO-A-01/47928
- WO-A-02/09713
- DE-A- 19 812 462
- BARATTI C M ET AL: "Effects of sildenafil on long-term retention of an inhibitory avoidance response in mice." BEHAVIOURAL PHARMACOLOGY. ENGLAND DEC 1999, Bd. 10, Nr. 8, Dezember 1999 (1999-12), Seiten 731-737, XP001097381 ISSN: 0955-8810 in der Anmeldung erwähnt
- SCHULTHEISS D ET AL: "COGNITIVE SIDE EFFECTS OF SILDENAFIL: AN ASSESSMENT USING EVENT-RELATED BRAIN POTENTIALS" EUROPEAN UROLOGY, S. KARGER AG., BASEL, CH, Bd. 37, Nr. SUPPL 2, März 2000 (2000-03), Seite 82 XP001041068 ISSN: 0302-2838
- GIULIANO F ET AL: "COMPARATIVE STUDY OF THE FACILITATOR PROERECTILE EFFECT OF VARDENAFIL AND SILDENAFIL IN ANAESTHETISED RATS" EUROPEAN UROLOGY, S. KARGER AG., BASEL, CH, Bd. SUPPL. 5, Nr. 39, März 2001 (2001-03), Seite 108,AN421 XP008005223 ISSN: 0302-2838
- SYBERTZ E ET AL: "Inhibitors of PDE1 and PDE5 cGMP phospodiesterases: patents and therapeutic potential" EXPERT OPINION ON THERAPEUTIC PATENTS, ASHLEY PUBLICATIONS, GB, Bd. 7, Nr. 6, 1997, Seiten 631-639, XP002178692 ISSN: 1354-3776

## Beschreibung

Die vorliegende Erfindung betrifft die Verwendung von 2-[2-Ethoxy-5-(4-methyl-piperazin-1-sulfonyl)-phenyl]-5-methyl-7-propyl-3*H*-imidazo[5,1-f][1,2,4]triazin-4-on bzw. dessen Hydrochlorid Trihydrat zur Herstellung eines Arzneimittels zur Verbesserung der Wahrnehmung, Konzentrationsleistung, Lernleistung und/oder Gedächtnisleistung, insbesondere zur Behandlung und/oder Prophylaxe von Störungen der Wahrnehmung, Konzentrationsleistung, Lernleistung und/oder Gedächtnisleistung.

Das cyclische Nucleotid cGMP (cyclisches Guanosinmonophophat) gehört zu den wichtigsten intrazellulären Botenstoffen und wird von bestimmten Phosphodiesterasen (PDEs), insbesondere dem Isoenzym PDE 5, metabolisiert *(Drugs Fut.* **2001**, *26*, 153-162). Die PDE 5 kommt vor allem in vaskulärem Glattmuskelzellgewebe, weniger in der Niere, Lunge und den Blutplättchen vor. Auf Grund ihrer vasorelaxierenden Wirkung wurden PDE 5-Inhibitoren zur Behandlung von Angina und Bluthochdruck, hauptsächlich aber zur Behandlung der erektilen Dysfunktion vorgeschlagen.

In der WO 99/24433 werden 2-Phenyl-substituierte Imidazotriazinone, ihre cGMP PDE-hemmende Wirkung sowie ihre Verwendung zur Behandlung von vaskulären Erkrankungen, insbesondere zur Behandlung der erektilen Dysfunktion, beschrieben.

In *Behav. Pharmacol.* **1999**, *10,* 731-737 wird die verbessernde Wirkung von Sildenafil auf die Erinnerungsfähigkeit von Mäusen in einem Vermeidungstest beschrieben.

Überraschenderweise wurde gefunden, dass sich 2-[2-Ethoxy-5-(4-methyl-piperazin-1-sulfonyl)-phenyl]-5-methyl-7-propyl-3*H*-imidazo[5,1-f][1,2,4]triazin-4-on bzw. dessen Hydrochlorid Trihydrat besonders gut zur Verbesserung der Wahrnehmung, Konzentrationsleistung, Lernleistung und/oder Gedächtnisleistung eignen.

Die Erfindung betrifft daher die Verwendung von 2-[2-Ethoxy-5-(4-methyl-piperazin-1-sulfonyl)-phenyl]-5-methyl-7-propyl-3*H*-imidazo[5,1-f][1,2,4]triazin-4-on bzw. dessen Hydrochlorid Trihydrat zur Herstellung eines Arzneimittels zur Verbesserung der Wahrnehmung, Konzentrationsleistung, Lernleistung und/oder Gedächtnisleistung.

2-[2-Ethoxy-5-(4-methyl-piperazin-1-sulfonyl)-phenyl]-5-methyl-7-propyl-3*H*-imidazo[5,1-f][1,2,4]triazin-4-on ist in der Tabelle A aufgeführt.

**Tabelle A:**

| **Struktur** |
|---|
| 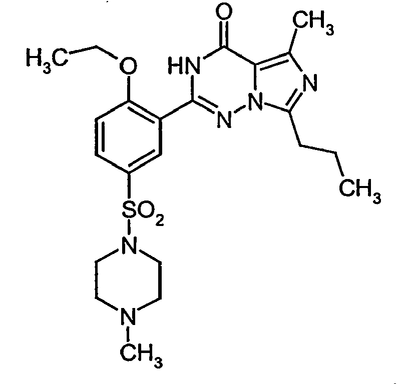 |

2-[2-Ethoxy-5-(4-methyl-piperazin-1-sulfonyl)-phenyl]-5-methyl-7-propyl-3*H*-imidazo[5,1-f][1,2,4]triazin-4-on bzw. dessen Hydrochlorid Trihydrat und ihre Herstellung sind in der WO 99/24433 beschrieben. Auf die Offenbarung der WO 99/24433 wird ausdrücklich Bezug genommen.

Eine weitere Ausführungsform der Erfindung betrifft die Verwendung von 2-[2-Ethoxy-5-(4-methyl-piperazin-1-sulfonyl)-phenyl]-5-methyl-7-propyl-3*H*-imidazo[5,1-f][1,2,4]triazin-4-on bzw. dessen Hydrochlorid Trihydrat zur Herstellung eines Arzneimittels zur Behandlung und/oder Prophylaxe von Störungen der Wahrnehmung, Konzentrationsleistung, Lernleistung und/oder Gedächtnisleistung, insbesondere wenn die Störung eine Folge von Demenz ist.

Besonders eignen sich die erfindungsgemäß verwendeten Verbindungen zur Verbesserung der Wahrnehmung, Konzentrationsleistung, Lernleistung, oder Gedächtnisleistung nach kognitiven Störungen, wie sie insbesondere bei Situationen/Krankheiten/Syndromen auftreten wie "Mild cognitive impairment", Altersassozüerte Lern- und Gedächtnisstörungen, Altersassozüerte Gedächtnisverluste, Vaskuläre Demenz, Schädel-Hirn-Trauma, Schlaganfall, Demenz, die nach Schlaganfällen auftritt ("post stroke dementia"), post-traumatisches Schädel Hirn Trauma, allgemeine Konzentrationsstörungen, Konzentrationsstörungen in Kindern mit Lern- und Gedächtnisproblemen, Alzheimersche Krankheit, Vaskuläre Demenz, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyolateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nichtüberzogene sowie überzogene Tabletten, z.B. magensaftresistente Überzüge), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Im Allgemeinen hat es sich als vorteilhaft erwiesen, bei parenteraler Applikation Mengen von etwa 0,001 bis 30 mg/kg, vorzugsweise etwa 0,01 bis 10 mg/kg Körpergewicht zur Erzielung wirksamer Ergebnisse zu verabreichen. Bei oraler Applikation beträgt die Menge etwa 0,01 bis 100 mg/kg, vorzugsweise etwa 0,1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Beispiel 1 ist 2-[2-Ethoxy-5-(4-methyl-piperazin-1-sulfonyl)-phenyl]-5-methyl-7-propyl-3*H*-imidazo[5,1-f][1,2,4]triazin-4-on und wird entsprechend dem Beispiel 16 in der WO 99/24433 hergestellt.

Beispiel 2 ist 2-[2-Ethoxy-5-(4-ethyl-piperazin-1-sulfonyl)-phenyl]-5-methyl-7-propyl-3H-imidazol[5,1-f][1,2,4]triazin-4-on Hydrochlorid-Trihydrat und wird entsprechend dem Beispiel 336 in der WO 99/24433 hergestellt.

Die PDE 5-inhibierende Wirkung der erfindungsgemäß verwendeten Verbindungen kann wie folgt bestimmt werden.

### Aktivität der PDE 5

Zur Testung der inhibierenden Wirkung wird der "Phosphodiesterase [³H] cGMP-SPA enzyme assay" der Firma Amersham Life Science verwendet. Der Test wird nach dem vom Hersteller angegebenen Versuchsprotokoll durchgeführt. Es wird humane rekombinante PDE 5 verwendet, die in einem Bacculovirussystem exprimiert wurde. Es wird die Substanzkonzentration gemessen, bei der die Reaktionsgeschwindigkeit um 50 % vermindert ist.

### Inhibition der PDEs 1 - 5 und 7

Rekombinante PDE1C (GenBank/EMBL Accession Number: NM_005020), PDE2A (Rosman et al. *Gene* **1997**, *191*, 89-95), PDE3B (Miki et al. *Genomics* **1996**, *36,* 476-485), PDE4B (Bolger et al. *Mol. Cell. Biol.* **1993**, 13, 6558-6571), PDE5A (GenBank/EMBL Accession Number: AJ004865) und PDE7B (Hetman et al. *Proc. Natl. Acad. Sci. U.S.A.* **2000**, *97*, 472-476) wurden mit Hilfe des pFASTBAC Baculovirus Expressionssystems (GibcoBRL) in Sf9 Zellen exprimiert.

Die *in vitro* Wirkung von Testsubstanzen an rekombinanter PDE3B, PDE4B, und PDE7B wird nach dem oben für PDE 10A beschriebenen Testprotokoll bestimmt. Für die Bestimmung einer entsprechenden Wirkung an rekombinanter PDE1C, PDE2A und PDE5A wird das Protokoll wie folgt angepaßt: Bei PDE1C werden zusätzlich Calmodulin 10⁻⁷ M und CaCl₂ 3mM zum Reaktionsansatz gegeben. PDE2A wird im Test durch Zugabe von cGMP 1 µM stimuliert und mit einer BSA Konzentration von 0,01 % getestet. Für PDE5A wird als Substrat [8-³H] guanosine 3', 5'-cyclic phosphate (Amersham Pharmacia Biotech., Piscataway, NJ) eingesetzt.

Die Beispiele 1 und 2 zeigen im PDE 5-Test IC₅₀-Werte von 0,6 bzw. 0,7 nM.

Die erfindungsgemäße Wirkung der Substanzen kann *in vivo* beispielsweise im Objekt-Wiedererkennungstest gezeigt werden.

### Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden.

Der Test wurde wie beschrieben durchgeführt (Blokland et al. *NeuroReport* **1998**, *9*, 4205-4208; Ennaceur, A., Delacour, J., *Behav. Brain Res.* **1988**, *31*, 47-59; Ennaceur, A., Meliani, K.,. *Psychopharmacology* **1992**, *109,* 321-330; Prickaerts, et al. *Eur. J. Pharmacol.* **1997,** *337*, 125-136).

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einem Intervall von 24 Stunden, wird die Ratte erneut in die Beobachtungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleichstark inspektieren. Die Gabe einer Substanz mit lern- und gedächtnisverbessernder Wirkung wird dazu führen, dass eine Ratte das bereits 24 Stunden vorher, im ersten Durchgang, gesehene Objekt als bekannt wiedererkennt. Sie wird das neue, unbekannte Objekt ausführlicher untersuchen als das bereits bekannte. Diese Gedächtnisleistung wird in einem Diskriminationsindex ausgedrückt. Ein Diskriminationsindex von Null bedeutet, dass die Ratte beide Objekte, das alte und das neue, gleichlang untersucht; d.h. sie hat das alte Objekt nicht wiedererkannt und reagiert auf beide Objekte als wären sie unbekannt und neu. Ein Diskriminationsindex größer Null bedeutet, dass die Ratte das neue Objekt länger inspektiert als das alte; d.h. die Ratte hat das alte Objekt wiedererkannt.

Die Effekte von Beispiel 1 auf die Objekt-Wiedererkennung von Ratten 24 Stunden nach dem ersten Durchgang wurden untersucht. Die Tiere erhielten oral Tylose alleine, oder Beispiel 1 in den Dosierungen 1,0, 3,0 oder 10 mg/kg Körpergewicht, suspendiert in Tylose, unmittelbar im Anschluss an den ersten Durchgang mit zwei identischen Objekten. Jeweils 24 Stunden später folgte der zweite Durchgang. Nach einer Auswaschperiode von 2 oder 3 Tagen wurde in denselben Ratten eine neue Dosierung von Beispiel 1 getestet, bis die Gedächtnisleistung aller Ratten zweimal in allen Dosierungen erfasst worden war. Alle Tiere dienten also als eigene Kontrolle. Die Resultate dieser Studie sind in Fig. 2 wiedergegeben. Überraschenderweise war die Gedächtnisleistung im zweiten Durchgang nach Behandlung mit 1,0, 3,0 oder 10 mg/kg von Beispiel 1 gegenüber der Kontrollbedingung (Behandlung mit Tylose alleine) verbessert. Der Diskriminationsindex war größer als Null und wich von dem in der Kontrollbedingung erreichten Diskriminationsindex ab.

## Patentansprüche

1. Verwendung von 2-[2-Ethoxy-5-(4-methyl-piperazin-1-sulfonyl)-phenyl]-5-methyl-7-propyl-3*H*-imidazo[5,1-f][1,2,4]triazin-4-on oder 2-[2-Ethoxy-5-(4-ethyl-piperazin-1-sulfonyl)-phenyl]-5-methyl-7-propyl-3H-imidazol[5,1-f][1,2,4]triazin-4-on Hydrochlorid-Trihydrat zur Herstellung eines Arzneimittels zur Verbesserung der Wahrnehmung, Konzentrationsleistung, Lernleistung und/oder Gedächtnisleistung.

2. Verwendung von Verbindungen nach Anspruch 1 zur Herstellung eines Arzneimittels zur Behandlung und/oder Prophylaxe von Störungen der Wahrnehmung, Konzentrationsleistung, Lernleistung und/oder Gedächtnisleistung.

3. Verwendung nach Anspruch 2, wobei die Störung eine Folge von Demenz ist.

## Claims

1. Use of 2-[2-ethoxy-5-[4-methylpiperazin-1-sulphoryl)phenyl]-5-methyl-7-propyl-3H-imidazo[5,1-f][1,2,4]triazin-4-one or 2-[2-ethoxy-5-(4-ethylpiperazin-1-sulphonyl)phenyl]-5-methyl-7-propyl-3H-imidazol[5,1-f][1,2,4]triazin-4-one hydrochloride trihydrate for the production of a medicament for improving the perception, concentration power, learning power and/or memory power.

2. Use of compounds according to Claim 1 for the production of a medicament for the treatment and/or prophylaxis of disorders of the perception, concentration power, learning power and/or memory power.

3. Use according to Claim 2, where the disorder is a result of dementia.

## Revendications

1. Utilisation de la 2-[2-éthoxy-5-(4-méthyl-pipérazin-1-sulfonyl)-phényl]-5-méthyl-7-propyl-3H-imidazo[5,1-f][1,2,4]triazin-4-one ou du trihydrate de chlorhydrate de 2-[2-éthoxy-5-(4-méthyl-pipérazin-1-sulfonyl)-phényl]-5-méthyl-7-propyl-3H-imidazo[5,1 -f][1,2,4]triazin-4-one pour la préparation d'un médicament pour l'amélioration de la perception, de la capacité de concentration, de la capacité d'apprentissage et/ou de la capacité de la mémoire.

2. Utilisation des composés selon la revendication 1 pour la préparation d'un médicament pour le traitement et/ou la prophylaxie de troubles de la perception, de la capacité de concentration, de la capacité d'apprentissage et/ou de la capacité de la mémoire.

3. Utilisation selon la revendication 2, dans lequel le trouble est la suite d'une démence.
